# EUROPEAN PATENT APPLICATION

(11) **EP 4 656 913 A1**
(43) Date of publication of application: **03.12.2025**
(21) Application number: 24179339.7
(22) Date of filing: 31.05.2024
(51) Int. Cl.: F16K 7/06, F16K 27/02, A61M 39/28

(54) **PINCH VALVE**

(71) Applicant: Sartorius Stedim Biotech GmbH, 37079 Göttingen (DE)
(72) Inventor: HEESE, Robin, 36179 Bebra (DE)
(74) Representative: Michalski Hüttermann & Partner Patentanwälte mbB

(57) **Abstract**

The invention relates to a pinch valve (2), including
a pinching member (10),
a base member (20) with a support section (22) facing the pinching member (10) and configured for receiving a tube (T), wherein
the pinching member (10) is movable relative to the base member (20) along a pinching direction (4) for pinching the tube (T), and
the support section (22) and/or the pinching member (10) comprises a recess (26) extending at least along the pinching direction (4) for receiving the tube (T) sectionally.

## Description

The invention relates to a pinch valve configured for receiving a tube and with a pinching member, wherein the pinching member is movable for pinching the tube. The invention furthermore relates to a method.

In chemical, pharmaceutical, biotechnological, medical and/or life science industries, media, e.g. fluids, is/are typically handled using tubes or hoses. In order to adjust a volume flow of the fluid though the tube or its pressure, pinch valves can be used for pinching the tube in order to reduce a cross section or cross sectional area thereof. A drawback of such known pinch valves is that an accurate control behavior, i.e. an accurate adjustment of volume flow and/or pressure, is very limited.

It is an object to provide solutions which enable a more secure and an easier handling of pinching a tube. Particularly, a more accurate control behavior is desired when pinching a tube. Particularly it is an object to avoid or reduce disadvantages of known solutions.

The object of the invention is solved by the features of the independent claims. Preferred implementations are detailed in the dependent claims.

Thus, the object is particularly solved by a pinch valve, including
a pinching member,
a base member with a support section facing the pinching member and configured for receiving a tube, wherein
the pinching member is movable relative to the base member along a pinching direction for pinching the tube, particularly with the pinching direction extending at least substantially in parallel to the tube and/or the base member, and
the support section and/or the pinching member comprises a recess particularly at least extending along the pinching direction for receiving the tube sectionally, preferably at least when the tube is pinched, particularly wherein the size, e.g. depth and/or width, of the recess is variable, e.g. decreasing or increasing, along the pinching direction and/or about the pinching member.

In other words, to give an example, the invention provides a pinching device configured for pinching a flexible tube inserted into the pinching device by moving a pinching member thereof relative to, especially substantially in parallel to, a counterpart, wherein at least one of pinching member and counterpart has a recess or groove which groove can sectionally receive the flexible tube. The pinching member may be moved substantially in parallel to the flexible tube. The grove may have a shape variable along its extension and/or a moving path of the pinching member.

The invention realizes a process-safe and user-friendly way of pinching the tube with a more accurate control behavior, since the tube can deform sectionally into the recess. The invention enables that the volume flow or pressure can be varied more homogeneously as a function of the movement of the pinching member particularly depending on the form or shape of the recess.

Relative to prior solutions that merely adopt a flat and/or recess-free support member or support surface for supporting the tube to be pinched, the recess makes it possible that the change in cross section of the pinched tube becomes more proportional to the movement of the pinching member.

The invention provides a possibility for reducing cost at little to no effect to accuracy. A manual movement of the pinching member can be conducted at higher accuracy. A motorized movement of the pinching member can be conducted at higher accuracy and/or with a less accurate or less expensive drive.

The invention may also realize that the tube may withstand more cycles of pinching.

The technical effects and advantages named herein may further enhance based on implementation of preferred or optional features as described herein.

The pinch valve particularly relates to a device configured for pinching a tube, e.g. in order to adjust or stop volume flow therethrough. The pinch valve typically is configured for squeezing and/or compressing the tube at least in a section. The pinch valve may reversibly reduce the cross section of the tube.

The tube, particularly when not pinched and/or when not deformed, typically has a round cross section. The tube can be deformed, e.g. is a flexible tube. The tube may comprise or consist of silicone and/or a polymer compound, such as a thermoplastic or an elastomer or a thermoplastic elastomer. The tube may comprise a braiding structure, such as mesh material, forming a composite with the polymer compound. The braiding structure may comprise a mesh material with polyethylene terephthalate, PET for short. The tube may have a Shore A hardness of at least 25 and/or up to 75, for example 50 ± 10 Shore A or 60 ± 10 Shore A. The tube may have a wall thickness of at least 1 mm and/or up to 6 mm, for example 1,5 mm ± 0,5 mm or 3 ± 0,5 mm or 3,25 ± 0,5 mm or 4,75 ± 0,5 mm. The tube may have an inner diameter of at least 2,5 mm and/or up to 36 mm, for example 3 mm ± 0,5 mm or 6,5 mm ± 0,5 mm or 9,5 mm ± 0,5 mm or 12,5 mm ± 0,5 mm or 19 mm ± 0,5 mm or 25,5 mm ± 0,5 mm.

The pinching member may be understood as a movable element or actuator of the pinch valve that can pinch the tube. The pinching member is movable along the pinching direction, typically at least sectionally along a straight line. The pinching direction may be understood as a path for the movement of the pinching member, which path may be, at least sectionally, straight and/or curved. It is preferred that the pinching member, at least sectionally, can be moved oblique or at least substantially in parallel to the tube to be pinched. The pinching member may additionally be rotatable, e.g. to roll on the tube during its movement.

The base member has the support section. The support section faces the pinching member. The support section and/or the recess may extend at least between an entry portion and an end portion. Alternatively or additionally, the recess may extend at least sectionally along and/or around the pinching member. The pinching member may be movable at least substantially between a first position, e.g. corresponding to the entry portion, and a second position, e.g. corresponding to the end portion. The pinching member may be movable beyond the first position and/or the second position. The first position may correspond to the tube being substantially not pinched and/or pinched lightly. The second position may correspond to the tube being pinched to stop fluid flow through the tube and/or to close the tube. Positions between the first and the second position may correspond to different reductions of the cross sectional area of the tube.

The support section is configured for receiving and/or supporting a tube that can be inserted into the pinch valve. The base member may be understood as a main body of the pinch valve on which or into which the tube can be placed. The tube can be received and supported by the support section. For example, the tube can be in contact to the support section, particularly to a support surface thereof and/or to the recess, in terms of area when inserted. The support section may be arranged on a side opposite the pinching member so that a gap or receptacle for the tube is formed between pinching member and base member. The support section may be understood as a section of the base member, e.g. at least a top surface thereof and/or a part thereof. The base member may have several sections.

The pinch valve may have and/or form a/the receptacle for the tube. For example, the receptacle may be formed by means of and/or between the base member and the pinching member. The receptacle may extend along or at least substantially in parallel to the pinching direction. The size of the receptacle may be adjustable, e.g. by adjusting a distance between the pinching member and the base member. Typically, said size of the receptacle or distance corresponds to the tube and/or is chosen between 1 and up to 20 mm, particularly between 3 and 15 mm, especially is 8 ± 5 mm.

The pinching member is movable relative to the base member. The pinching member can be moved along the pinching direction for pinching the tube. The pinching direction can extend substantially in parallel to the tube. For example, the pinching member may be guided by means of the base member. The pinching member may be movable along the support section, particularly substantially in parallel thereto. The pinching member may comprise a face configured for contacting the tube, e.g. an at least sectionally flat and/or curved face.

The support section and/or the pinching member comprises the recess, particularly wherein the recess extends at least along the pinching direction and/or about an axis of the pinching member. Particularly, the recess is meant and/or configured for receiving the tube sectionally. The recess may comprise or consist of a groove and/or may extend in the base member and/or in the pinching member. The recess may have an elongated form, e.g. elongated at least in sections along and/or in parallel to the pinching direction. The recess may have a width extending perpendicular to the pinching direction and/or to the axis and a length extending along the pinching direction and/or around the axis and particularly a depth extending perpendicular to the width and the length and/or perpendicular to the axis. The length may be greater than the width and/or the depth, particularly greater by the factor of two, four or more.

Particularly, the recess may have a shape, depth and/or width, variable along the pinching direction and/or along the length and/or about the pinching member. Particularly as an average, the depth and/or width may be at least 1 mm or at least 5 mm or at least 7,5 mm. The depth and/or width of the recess or average value(s) may be considered at the entry portion, the end portion or between the entry portion and the end portion, e.g. substantially in the middle therebetween.

The recess may be understood as a kerf or groove. The recess may be large enough to receive at least a section of the tube, particularly that is deformed and/or compressed e.g. when being pinched. The recess may be less wide than a width, outer diameter and/or inner diameter of the tube, at least at its widest point and/or at the entry portion. Typically, the largest width and/or depth is provided at the entry portion. The recess may be shaped to sectionally surround the tube.

The support section may comprise a support surface for supporting the tube at least sectionally, wherein the support surface may extend along and/or in parallel to the pinching direction. The support surface may surround the recess of the support section at least sectionally. The support surface may be at least substantially flat and/or curved particularly extending along or in parallel to the pinching direction. The support surface may be a top surface of the base member. The support surface may be wider than a width, outer diameter and/or inner diameter of the tube, at least at its widest point. In some embodiments, the recess and the support surface may form a smooth and/or fuzzy transition between each other, e.g. by means of a transition with a chamfered, inclined convex and/or concave form.

The base member may have an inclined section. The inclined section may be arranged adjacent to the support section along the pinching direction at an/the entry portion of the support section. For example, the inclined section may in principle form an obtuse angle with the support section and/or support surface. A transition between the inclined section and the support section may include a step or chamfered step. The inclined section and the support section may form a smooth and/or fuzzy transition between each other, e.g. by means of a transition with a chamfered, inclined convex and/or concave form. This protects the tube mechanically from damage.

The recess may have a depth variable at least along the pinching direction and/or around the pinching member. For example, the depth may be different at different positions along the pinching direction and/or around the pinching member in order to affect the size of the section of the tube that can be received by the recess. Particularly, the depth may decrease from an/the entry portion towards an/the end portion of the support section. The recess may be inclined and/or run upwards, particularly from the entry portion towards the end portion. The recess may substantially and/or at least sectionally follow a straight line and/or a curved line. Particularly, in the end portion, the depth may be substantially zero and/or the support surface may be flat. With such a design the movement of the pinching member results in that a relatively large movement of the pinching member is possible for a very accurate adjustment of the pinching. Furthermore, differently sized tubes can be handled. Also, a calibration of the pinch valve or sophisticated placement of the tube may possibly be omitted.

The recess may have a width variable at least along the pinching direction and/or around the pinching member. The width may decrease from an/the entry portion towards an/the end portion of the support section. For example, the width may be different at different positions along the pinching direction in order to affect the size of the section of the tube that can be received by the recess. For example, depth and width may both decrease in the same direction. Particularly, in the end portion, the width may be substantially zero and/or the support surface may be flat. The width may be defined between two borders of the recess and/or adjacent to the support surface, particularly wherein one or both of the borders may substantially and/or at least sectionally follow a straight line and/or a curved line. An accurate adjustment of the pinching is thus possible.

The recess at least sectionally may have a curved or circular shape in a cross section with respect to the pinching direction. For example, the recess may have a cylindrical and/or round and/or semi-circular shape or contour, particularly a concave shape or contour. Such a shape results in a smooth deformation of the tube and an easy manufacture of the base member.

The recess at least sectionally may have a U-shape in a cross section with respect to the pinching direction, particularly the U-shape having a rectangular, quadratic or trapezoidal contour. The U-shape may have two opposing flanks and a base between the two flanks. The flanks may be at least substantially parallel or oblique to each other. The flanks, particularly in the case of a trapezoidal contour, may provide a wider top and a narrower bottom of the groove. The flanks may form an angle, particularly of at least 0° and/or up to 170°, particularly the angle is 10° ± 10°. The base at least substantially may be perpendicular to one or both of the flanks.

The recess at least sectionally may have a V-shape in a cross section with respect to the pinching direction. The V-shape may have two opposing flanks that are oblique to each other. The flanks may form a particularly acute angle. The angle between the flanks may be at least 10° and/or up to 170°, particularly the angle is 90° ± 25°.

The pinching member may comprise a roll. Preferably, the pinching member and/or roll is configured to rotate about an axis oblique or perpendicular to the pinching direction. The pinching member and/or roll may be configured to rotate about the axis substantially in parallel to the support surface. Said axis may be guided along the pinching direction, particularly obliquely or perpendicularly thereto. The pinching member and/or roll may comprise a cylindrical and/or round shape or contour. The outer diameter of the roll may be at least 5 mm and/or up to 50 mm. The roll may reduce friction on the tube. The roll may reduce mechanical load to the tube.

The recess may be formed on the roll, particularly the recess surrounding the roll at least partially or fully about the axis. The recess may be formed around the roll, thus extending about the roll and optionally, at least sectionally, along the pinching direction as well as in other directions. The recess may have a depth and/or width variable about the axis of the pinching member, e.g. variable as a function of an angular position of the pinching member.

The pinch valve may have a guidance member. The guidance member may have an opening or clearance with a form corresponding to the pinching direction and/or desired path of movement of the pinching member. The guidance member, particularly the opening or clearance, may extend along the pinching direction and may be configured for guiding the pinching member, particularly for guiding the pinching member at least sectionally along a straight line.

The guidance member may be coupled to and/or formed with the base member. For example, the guidance member may have at least one guidance part configured for guiding the pinching member by means of an opening thereof extending along the pinching direction. The guidance member may comprise (the) guidance parts each of which can be arranged on one side of the pinching member, particularly wherein particularly at least one of the guidance parts can be removably attached to the base member for insertion of the tube. The guidance parts may have a cut-out or groove, e.g. the opening or clearance, corresponding to the pinching direction.

The pinching member, the base member and/or the guidance member may comprise or consist of a polymer compound and/or a metal alloy. Polymer compounds are cheap, lightweight and relatively robust. Metal alloys, such as aluminum or steel, may be more expensive and less lightweight, but more robust than the polymer compound. A combination of polymer compound and metal alloy may be considered for different parts or sections of the pinch valve. For example, a mount or axis of the pinching member and/or the pinching member and/or the guidance member may comprise or consist of metal. The support member may comprise or consist of polymer compound.

The pinching member may be meant for manual movement. The pinch valve may alternatively or additionally comprise a drive member configured for moving the pinching member. The drive member may be pneumatically or electrically driven. The drive member may comprise a stepper motor or linear drive. The drive member may be coupled to the pinching member and configured for movement along the pinching direction.

The drive member may be configured to rotate the pinching member, especially the roll having the recess. The drive member may be configured to rotate the roll for the roll to assume specific angular positions corresponding to a shape of the recess on the roll at the specific angular positions to control pinching of the tube.

The pinch valve may comprise a control arrangement configured to control the drive member for controlling a fluid flow through the tube, especially by means of moving the pinching member. The control arrangement may be configured for considering a measured fluid flow through the tube in order to control the fluid flow.

The object is further solved by means of a method for pinching a/the tube by means of the pinch valve, comprising the steps of
arranging the tube between the pinching member and the base member with the tube extending along the pinching direction and received by the base member,
moving the pinching member along the pinching direction for pinching the tube, wherein the tube is being compressed and/or deformed and is being sectionally received by the recess.

The method may include removing and/or attaching a guidance member, particularly a guidance part of the guidance member, to the base member particularly in order to arrange the tube laterally and/or sideways into the pinch valve. Moving may be conducted by means of a drive member, e.g. electrically or pneumatically. The method may include that a fluid is flowing through the tube, wherein moving the pinching member results in a change in cross sectional area of the tube in order to reduce a fluid volume flow and/or fluid pressure.

The object is further solved by means of a system including the pinch valve and the tube.

These and other aspects of the invention will be apparent from and elucidated with reference to the implementations described hereinafter. Whenever 'or' is used in the present disclosure, 'or' may be replaced by 'and/or'. A 'member' may relate to a singular part or to a multi-part assembly.

In the drawings:
Fig. 1A-C shows a pinch valve in a perspective view;
Fig. 2 shows a base member of a pinch valve in a perspective view;
Fig. 3 shows a base member of a pinch valve in a perspective view;
Fig. 4 shows a pinch valve pinching a tube in a perspective view;
Fig. 5A-D shows a pinch valve pinching a tube in a side view and in a sectional view in four different positions of a pinching member of the pinching valve;
Fig. 6A-B shows a pinching member of a pinch valve in a frontal view (A) and in a cross sectional view (B);
Fig. 7A-B shows a second pinching member of a pinch valve in a frontal view (A) and in a cross sectional view (B); and
Fig. 8A-B shows a third pinching member of a pinch valve in a frontal view (A) and in a cross sectional view (B).

The description contains procedural or methodical aspects upon describing structural features; the structural features can be understood well in that way. It is emphasized to the reader that such structural features can be lifted from the described context without hesitation or the question of an intermediate generalization to form aspects of the invention. It is also emphasized to the reader that any the structural features described in the following can be understood as individual aspects of the invention to distinguish from known solutions, despite being possibly lifted from the context.

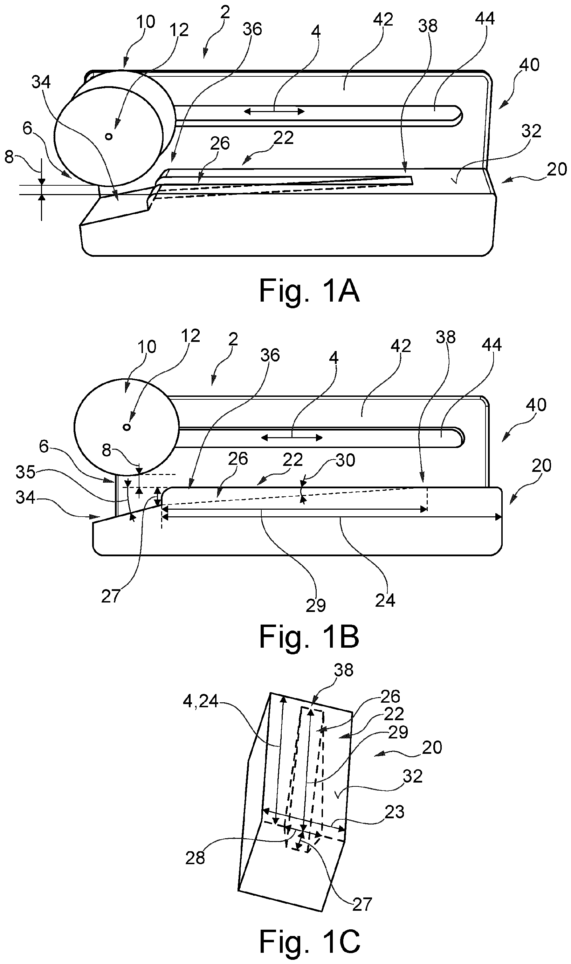

Fig. 1A-B shows a pinch valve 2 including a pinching member 10, a base member 20 with a support section 22 facing the pinching member 10 and configured for receiving a tube T (not shown) that can be inserted into the pinch valve 2. Fig. 1C shows the base member 20 only.

The pinching member 10 is movable relative to the base member 20 along a pinching direction 4 for pinching the tube T. The pinching direction 4 extends along a straight line. The pinching member 10 comprises a roll that is configured to rotate about an axis 12 perpendicular to the pinching direction 4 and in parallel to the support surface 32. The pinching member 10 is arranged at a distance 8 to the base member 20 and particularly forms with the base member 20 a receptacle 6 for the tube T. The distance 8 may be at least 1 mm and/or up to 10 mm, e.g. the distance 8 may be adjustable.

The pinch valve 2 comprises a guidance member 40 with two guidance parts 42 each of which is arranged on one side of the pinching member 10 and particularly coupled thereto. One of the guidance parts 42 is removably attached to the base member 20 for insertion of the tube T, wherein said one of the guidance parts is hidden in the present illustration. Each of the guidance parts 42 has an opening 44. The guidance member 40 extends along the pinching direction 4. By means of the openings 44, the guidance member 40 is configured for guiding the pinching member 10 along a straight line.

The pinching member 10, the base member 20 and the guidance member 40 comprise at least one polymer compound.

The pinch valve 2 has a pneumatically or electrically driven drive member configured for moving the pinching member 10 particularly along the pinching direction 4.

Typically, the pinching member 10 or its roll can rotate freely. Optionally, the pinching member 10 or its roll may be driven to rotate e.g. about the axis 12.

The support section 22 comprises a recess 26 extending along the pinching direction 4 for receiving the tube T sectionally. The support section 22 comprises a support surface 32 extending along the pinching direction 4 for supporting the tube T sectionally. As can be seen in Fig. 1A, the support surface 32 surrounds the recess 26 at least sectionally. The support surface 32 is flat and extends in parallel to the pinching direction 4.

The base member 20 has an inclined section 34 that is arranged adjacent to the support section 22 along the pinching direction 4 at an entry portion 36 of the support section 22. The inclined section 34 is arranged at an angle 35 relative to the pinching direction 4, particularly the angle 35 being between 5° and 60°, in this case the angle 35 being 15° ± 7,5°.

A transition between the inclined section 34 and the support section 22 is chamfered and/or rounded. Presently, the transition between the inclined section 34 and the support section 22 includes a step.

The recess 26 extends between the entry portion 36 and an end portion 38 of the support section 22. A length 29 of the recess 26 extends between the entry portion 36 and the end portion 38. The length 29 of the recess 26 is less than a length 24 of the support section 22. A width 28 of the recess is less than a width 23 of the base member 20.

With respect to Fig. 1A-C, the recess 26 has a depth 27 variable along the pinching direction 4. The depth 27 decreases, particularly substantially linearly, from the entry portion 36 towards the end portion 38. In the end portion 38, the depth 27 is substantially zero and the support surface 32 is flat. The recess 26 is arranged at an angle 30 relative to the pinching direction 4, particularly the angle 30 being between 0° and 20°, in this case the angle 35 being 5° ± 2,5°.

With respect to Fig. 1A-C, the recess 26 has a width 28 variable along the pinching direction 4. The recess 26 has a U-shape in a cross section with respect to the pinching direction 4. the U-shape has a trapezoidal contour. The width 28 decreases, particularly substantially linearly, from the entry portion 36 towards the end portion 38 of the support section 22. The width 28 is considered at the border with the support surface 32. The decrease in width 28 along the base member 20 in this case results from the trapezoidal contour being a substantially tilted form removed from the base member 20.

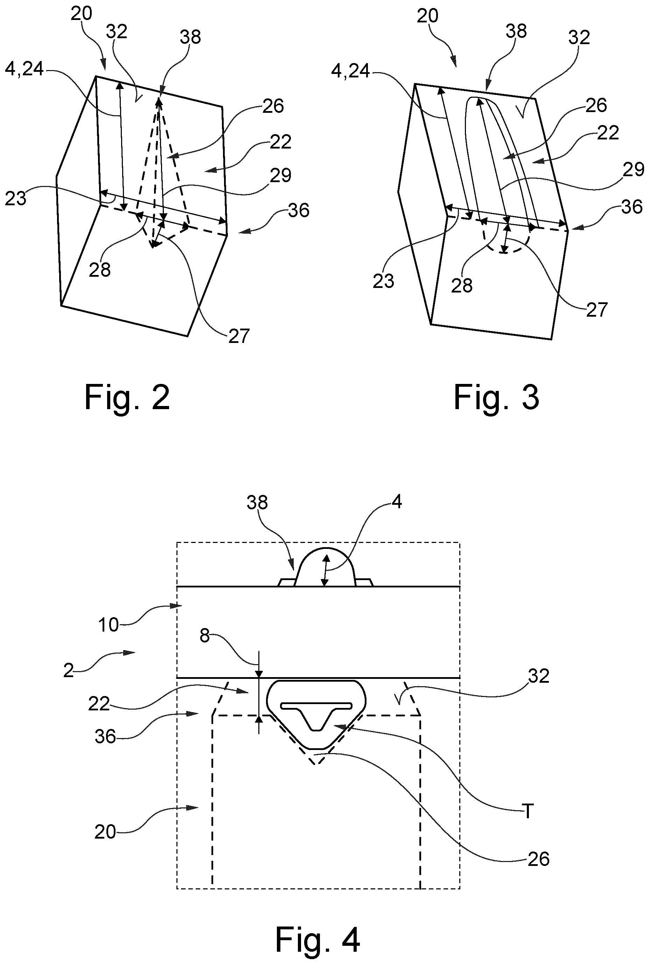

Figs. 2 and 3 show further embodiments of base members 20 with a recess 26, a flat support surface 32, a length 24 and a width 23. In each embodiment, the recess 26 extends along a pinching direction 4 between an entry portion 36 and an end portion 38. A length 29 of the recess 26 extends between the entry portion 36 and the end portion 38. The length 29 of the recess 26 is less than a length 24 of the support section 22.

With respect to Fig. 2, the recess 26 has a depth 27 variable along the pinching direction 4. The depth 27 decreases, particularly substantially linearly, from the entry portion 36 towards the end portion 38. In the end portion 38, the depth 27 is substantially zero and the support surface 32 is flat. The recess 26 is arranged at an angle relative to the pinching direction 4 or the support surface 32.

With respect to Fig. 2, the recess 26 has a width 28 variable along the pinching direction 4. The recess 26 has a V-shape in a cross section with respect to the pinching direction 4. In the cross section, the V-shape makes up an angle between 40° and 180°, in this case the angle being 45° ± 10°. The width 28 decreases, particularly substantially linearly, from the entry portion 36 towards the end portion 38 of the support section 22. The width 28 is considered at the border with the support surface 32. The decrease in width 28 along the base member 20 in this case results from the V-shape being a substantially tilted form removed from the base member 20.

With respect to Fig. 3, the recess 26 has a depth 27 variable along the pinching direction 4. The depth 27 decreases, particularly substantially linearly, from the entry portion 36 towards the end portion 38. In the end portion 38, the depth 27 is substantially zero and the support surface 32 is flat. The recess 26 is arranged at an angle relative to the pinching direction 4 or the support surface 32.

With respect to Fig. 3, the recess 26 has a width 28 variable along the pinching direction 4. The recess 26 has a curved and/or circular shape in a cross section with respect to the pinching direction 4. The width 28 decreases, particularly substantially linearly, from the entry portion 36 towards the end portion 38 of the support section 22. The width 28 is considered at the border with the support surface 32. The decrease in width 28 along the base member 20 in this case results from the curved and/or circular shape being a substantially tilted form removed from the base member 20. The border thus has a curved and/or ellipsoidal shape. The transition between the recess 26 and the support surface 32 is chamfered and/or rounded.

Fig. 4 shows a frontal view on a tube T inserted into a pinch valve 2 and between a pinching member 10 and a base member 20 of the pinch valve 2. The pinching member 10 is arranged at a distance 8 to the base member 20, particularly to its support surface 32, which distance 8 is smaller than the outer diameter of the tube T. The distance 8 may be substantially constant when the pinching member 10 is moved relative to the base member 20 along a pinching axis 4, the recess 26 and/or the support surface 32.

The base member 20 has a support section 22 facing the pinching member 10 which support section 22 receives the tube T. The base member 20 may be that of Fig. 2, i.e. comprising a recess 26 with a V-shape. The tube T is at least sectionally received by the recess 26. In the present illustration, the pinching member 10 is arranged between an entry portion 36 and an end portion 38 to be moved therebetween.

Depending on the position of the pinching member 10, the tube T may be sectionally supported by the support surface 32, by the recess 26 or by both. In other words, for example, the tube T can rest on the support surface 32 or on the surface of the recess 26 or on both. In Fig. 4, the tube T is supported and particularly sectionally received by the recess 26.

Upon further movement of the pinching member 10 along the pinching direction 4 towards the end portion 38 the depth and width of the recess 26 decrease so that the pinching member 10 may pinch the tube T more. This may successively result in a reduction of a possible volume flow through the tube T.

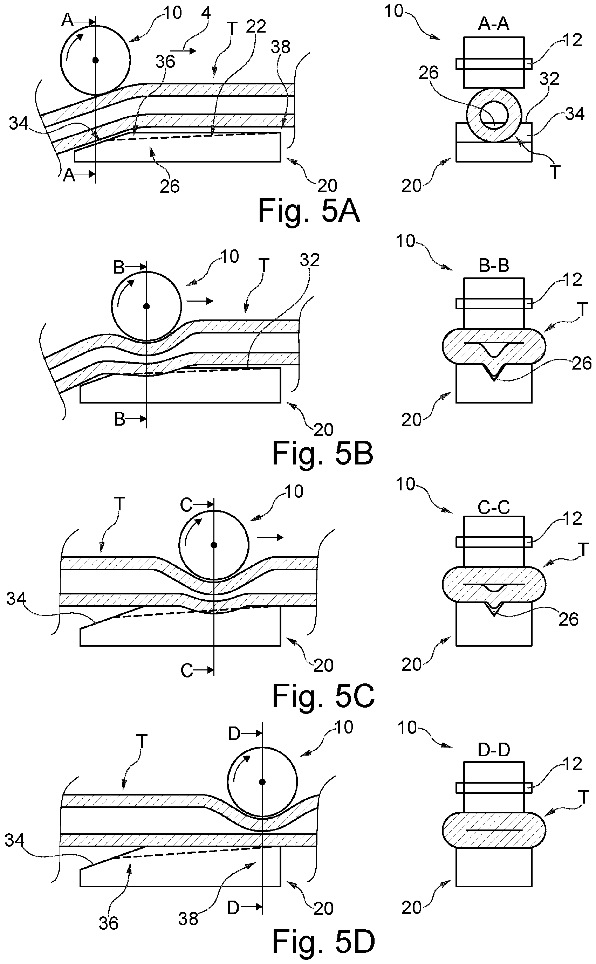

Fig. 5A-D schematically discloses a method for pinching a tube T by means of a pinch valve 2, such as the pinch valve 2 of Fig. 1A-D. From top to bottom, Fig. 5A-D shows four states of pinching the tube T, wherein each of the four states can be seen from a side (left) and in a section (right). The states substantially relate to different positions of a pinching member 10.

In a first state (Fig. 5A and section A-A), the tube T is not pinched wherein the cross sectional area is substantially round. In a fourth state (Fig. 5D), the tube T is fully pinched making its cross sectional area for fluid flow zero. A second state (Fig. 5B) and a third state (Fig. 5C) correspond to the tube T being pinched to reduce its cross sectional area for fluid flow.

The method includes arranging the tube T between a pinching member 10 and a base member 20 of the pinch valve 2, wherein the tube T extends along a pinching direction 4 and is received by the base member 20. The pinching direction 4 relates to the direction along which the pinching member 10 is movable. The base member 20 has a support section 22 including a recess 26 extending along the pinching direction 4 between an entry portion 36 and an end portion 38 and a flat support surface 32 surrounding the recess 26. The base member 20 also has an inclined section 34 arranged adjacent to the support section 22 along the pinching direction 4. The tube T may rest on the inclined section 34, cf. e.g. Fig. 5A.

The recess 26 becomes more narrow and shallower along the pinching direction 4, in Fig. 5A-D towards the right as indicated with a dotted line.

As illustrated throughout the four states shown in Fig. 5A-D, the method includes moving pinching member 10 along the pinching direction 4 for pinching the tube T, wherein the tube T is being compressed and is being sectionally received by the recess 26. Upon moving the pinching member 10, the pinching member 10 can rotate freely about its axis 12, particularly is not driven with respect to its rotation, and can roll along the tube T, cf. the circular arrow of the pinching member 10. The pinching member 10 may be moved by a stepper motor or linear drive.

With respect to Fig. 5A-D, the pinching member 10 can be moved between a first position (Fig. 5A) and a second position (Fig. 5D). The first position corresponds to the tube T being substantially not pinched and/or pinched lightly. The second position corresponds to the tube T being pinched to stop fluid flow through the tube T and/or to close the tube T. Positions between the first and the second position (Fig. 5B and 5C) corresponds to different reductions of the cross sectional area of the tube T.

Particularly as depicted in Fig. 5B and Fig. 5C, the tube T is sectionally received in the recess 26. The cross section in the tube T becomes lower in size upon moving the pinching member 10 further towards an end of the recess 26 and/or a shallower region of the recess 26 and/or an end portion 38. It can be seen that the recess 26 serves as a retreat volume for the tube T to be deformed into so that the volume flow through the tube T can be adjusted accurately as a function of the position of the pinching member 10. Due to the movement of the pinching member 10 it presses the tube T into a forced position (predetermined contour) in the recess 26 of the counterpart, namely the base member 20. A defined opening arises in the center of the tube T in accordance with the recess 26. The sides of the tube T may be closed, cf. section B-B or C-C.

As illustrated in Fig. 5D and particularly section D-D, the tube T is fully compressed and/or closed so that no fluid can pass the tube T.

The pinching member 10 may be adjustable towards and/or away from the base member 10, e.g. to adapt to differently sized tubes T.

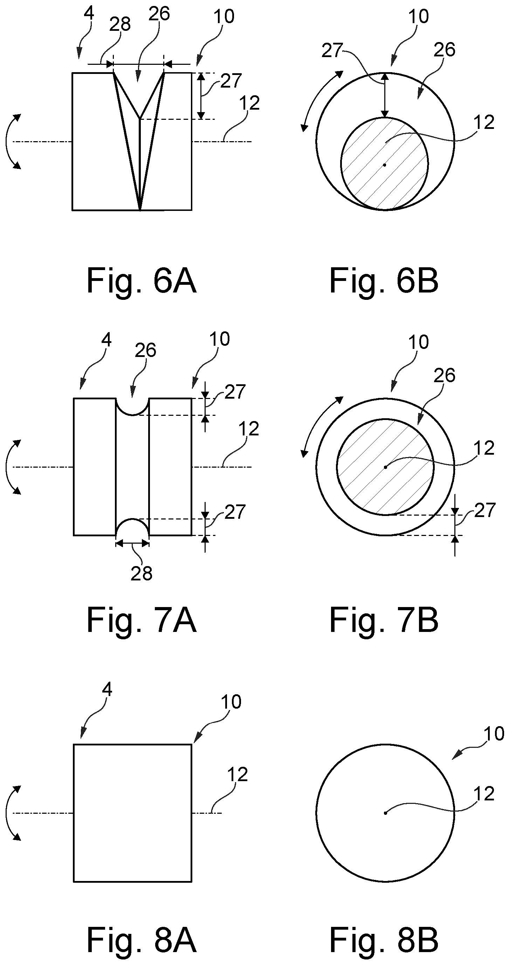

Fig. 6A-B shows a pinching member 10 that comprises a recess 26 for receiving a tube T sectionally. The recess 26 is formed on a roll of the pinching member 10 that is configured to rotate about an axis 12. The recess 26 extends at least along a pinching direction 4 and about the axis 12. Particularly, the recess 26 surrounds the roll at least partially, and in this case almost fully, about the axis 12. Particularly, in at least one section the recess 26 comprises a depth of substantially zero or is not present. It can for example be seen that the recess 26 is not present in a first section that is in this case opposite a second section.

The recess 26 has a depth 27 variable along the pinching direction 4 and about the axis 12 of the roll. The recess 26 has a width 28 variable along the pinching direction 4 and/or about the axis 12. The recess 26 has a V-shape in a longitudinal section with respect to the axis 12 or cross section with respect to a pinching direction 4. The pinching member 10 may be used in combination with a base member 20 having a support section 22 that has a recess 26 or that does not have a recess 26, e.g. has a substantially flat support section 2. The pinching member 10 moved along the pinching direction 4 and rotating while in contact with the tube T can provide that the tube T is pinched as a function of the angular position of the roll, since the depth 27 of the recess varies accordingly. For example, the pinching member 10 may be used to replace the pinching member of the pinch valve 2 shown in Fig. 1A-B. Particularly in this example, the base member 20 may not have the recess 26.

The pinching member 10 of Fig. 6A-B may be used in combination with a drive member that is configured to rotate the pinching member 10 about the axis 12 and particularly to move the pinching member along the pinching direction 4. The drive member may be configured to rotate the roll for the roll to assume specific angular positions corresponding to a shape of the recess 26 on the roll at the specific angular positions to control pinching of the tube T. In other words, particularly, the drive member can enable it to rotate the roll in line with the recess depth 27 in relation to the lateral movement along pinching direction 4 and rolling circumference of the roll.

Fig. 7A-B shows another pinching member 10 that comprises a recess 26 for receiving a tube T sectionally. The recess 26 is formed on a roll of the pinching member 10 that is configured to rotate about an axis 12. The recess 26 extends at least along a pinching direction 4 and about the axis 12. Particularly, the recess 26 surrounds the roll at least partially, and in this case fully, about the axis 12.

The recess 26 has a depth 27 that is substantially constant along the pinching direction 4 and about the axis 12 of the roll. The recess 26 has a width 28 that is substantially constant along the pinching direction 4 and/or about the axis 12. The recess 26 has a U-shape or curved shape in a longitudinal section with respect to the axis 12 or cross section with respect to the pinching direction 4. The pinching member 10 may be used in combination with a base member 20 having a support section 22 that has a recess 26 or that does not have a recess 26. For example, the pinching member 10 may be used to replace the pinching member of the pinch valve 2 shown in Fig. 1A-B. Particularly in this example, the base member 20 may have the recess 26.

Fig. 8A-B shows a third pinching member 10 with a roll having a cylindrical contour. The pinching member 10 is configured to rotate about an axis 12. Such a pinching member 10 may be the pinching member 10 as shown in Fig. 1A-B and/or in Fig. 5A-D.

In Fig. 6-8, the axis 12 is perpendicular to the pinching direction 4.

### Reference signs list

- 2: pinch valve
- 4: pinching direction
- 6: receptacle
- 8: size
- 10: pinching member
- 12: axis
- 20: base member
- 22: support section
- 23: width of 20
- 24: length of 20
- 26: recess
- 27: depth of 26
- 28: width of 26
- 29: length of 26
- 30: angle between 26 and 4
- 32: support surface
- 34: inclined section
- 35: angle between 34 and 4
- 36: entry portion
- 38: end portion

- 40: guidance member
- 42: guidance part
- 44: opening

- T: tube

## Claims

1. Pinch valve (2), including
a pinching member (10),
a base member (20) with a support section (22) facing the pinching member (10) and configured for receiving a tube (T), wherein
the pinching member (10) is movable relative to the base member (20) along a pinching direction (4) for pinching the tube (T), and
the support section (22) and/or the pinching member (10) comprises a recess (26) extending at least along the pinching direction (4) for receiving the tube (T) sectionally.

2. Pinch valve (2) according to claim 1, wherein
the support section (22) comprises a support surface (32) extending along the pinching direction (4) for supporting the tube (T) at least sectionally, and preferably
the support surface (32) surrounds the recess (26) at least sectionally.

3. Pinch valve (2) according to according to any one of the preceding claims, wherein the base member (20) has an inclined section (34) arranged adjacent to the support section (22) along the pinching direction (4) at an entry portion (36) of the support section (22).

4. Pinch valve (2) according to according to any one of the preceding claims, wherein the recess (26) has a depth (27) variable at least along the pinching direction (4).

5. Pinch valve (2) according to according to the preceding claim, wherein the depth (27) decreases from an/the entry portion (36) towards an end portion (38) of the support section (22), wherein, in the end portion (38), the depth (27) is substantially zero and/or the support surface (32) is flat.

6. Pinch valve (2) according to according to any one of the preceding claims, wherein the recess (26) has a width (28) variable at least along the pinching direction (4), optionally wherein the width (28) decreases from an/the entry portion (36) towards an/the end portion (38) of the support section (22).

7. Pinch valve (2) according to according to any one of the preceding claims, wherein the recess (26) at least sectionally has a curved or circular shape in a cross section with respect to the pinching direction (4).

8. Pinch valve (2) according to according to any one of the preceding claims,
wherein the recess (26) at least sectionally has a U-shape in a cross section with respect to the pinching direction (4), particularly the U-shape having a rectangular or trapezoidal contour, or
wherein the recess (26) at least sectionally has a V-shape in a cross section with respect to the pinching direction (4).

9. Pinch valve (2) according to according to any one of the preceding claims, wherein the pinching member (10) comprises a roll that is configured to rotate about an axis (12) oblique or perpendicular to the pinching direction (4) and particularly in parallel to the support surface (32), particularly wherein the recess (26) is formed on the roll, the recess (26) surrounding the roll at least partially or fully about the axis (12).

10. Pinch valve (2) according to according to any one of the preceding claims, comprising a guidance member (40) extending along the pinching direction (4) and configured for guiding the pinching member (10), particularly for guiding the pinching member (10) at least sectionally along a straight line.

11. Pinch valve (2) according to according to the preceding claim, wherein the guidance member (40) is coupled to and/or formed with the base member (20).

12. Pinch valve (2) according to according the preceding claim, the guidance member (40) comprising guidance parts (42) each of which is arranged on one side of the pinching member (10), particularly wherein one of the guidance parts (42) is removably attached to the base member (20) for insertion of the tube (T).

13. Pinch valve (2) according to according the preceding claim, wherein the pinching member (10), the base member (20) and/or the guidance member (40) comprises or consists of a polymer compound and/or a metal alloy.

14. Pinch valve (2) according to according the preceding claim, comprising a drive member (50) configured for moving the pinching member (10) and pneumatically or electrically driven.

15. Method for pinching a tube (T) by means of a pinch valve (2) according to any one of the preceding claims, comprising the steps of
arranging the tube (T) between the pinching member (10) and the base member (20) with the tube (T) extending along the pinching direction (4) and received by the base member (20),
moving the pinching member (10) along the pinching direction (4) for pinching the tube (T), wherein the tube (T) is being compressed and is being sectionally received by the recess (26).
